# EUROPEAN PATENT APPLICATION

(11) **EP 4 663 774 A1**
(43) Date of publication of application: **17.12.2025**
(21) Application number: 25181640.1
(22) Date of filing: 10.06.2025
(51) Int. Cl.: C12Q 1/6883

(54) **IN VITRO PROGNOSTIC METHOD**

(30) Priority: 11.06.2024 IT 202400013375
(71) Applicant: Fondazione IRCCS Ca' Granda - Ospedale Maggiore Policlinico, 20122 Milano (IT); UNIVERSITA' DEGLI STUDI DI MILANO, 20122 Milano (IT)
(72) Inventor: FENOGLIO, Chiara, I-20122 Milan (IT); GALIMBERTI, DANIELA, I-20122 Milan (IT); SCARPINI, ELIO ANGELO, I-20122 Milan (IT); SERPENTE, MARIA, I-20122 Milan (IT)
(74) Representative: Merli, Silvia

(57) **Abstract**

It forms an object of the present invention to provide a method for early predictive diagnosis of dementia associated with neurodegenerative pathology, where said method includes:
- Make available a plasma sample from at least one test subject;
- Isolate neuronal extracellular vesicles (NDEVs) in said sample;
- Isolate miRNAs contained in said NDEVs;
- Proceed with a quantitative amplification of miR-190b-5p contained in said miRNAs isolated from said NDEVs and miR-190b-5p contained in a population of miRNAs made available as a control, where levels of miR-190b-5p measured in said miRNAs from said subject in analysis greater than the levels of miR-190b-5p measured in the control are indicative of a forthcoming onset of neurodegeneration-associated dementia.

## Description

### State of the Art.

Extracellular vesicles (EVs) are composed of a heterogeneous collection of particles between 50 and 500 nm in diameter, released from almost all cells under physiological and pathological conditions, and play a key role in intercellular communication.

Significant is their role in the central nervous system (CNS), because of their ability to connect neurons and glia, and CNS with the rest of the body, as they are able to cross the blood-brain barrier. EVs, also released from neurons (NDEVs, *Neural Derived Extracellular Vesicles*) are thus a good representation of the CNS, which can be detected in peripheral blood.

EVs have a demonstrated role in Alzheimer's disease (AD). As an example, Joshi P et al. 2014, Cell Death & Differ.21: 582-93 showed elevated levels of EVs from microglia in the peripheral blood of dementia patients.

In addition, the contents of EVs include micro(mi)RNAs, small noncoding RNAs that play a role as post transcriptional regulators. Given the stability of miRNAs in biological fluids, and their massive presence in EVs that cross the blood-brain barrier and reach the peripheral circulation, miRNAs are considered allies in the search for biomarkers for neurodegenerative diseases

Gámez-Valero et al., investigates the microRNA profiles associated with total plasma-derived extracellular vesicles (EVs) in patients AD, dementia with Lewy bodies (DLB), and healthy controls. They identified specific microRNAs (i.e. miR-451a and miR-21-5p) that are significantly downregulated in AD compared to DLB and controls.

To date, the diagnosis of AD and frontotemporal dementia (FTD), the most common forms of dementia, is made exclusively when cognitive decline is already underway, an irreversible situation in which neurons are already compromised.

This explains the lack of effective treatments, as even innovative therapies cannot work on brain cells that are already dead or so severely damaged that they cannot be recovered

Early diagnosis of dementia would be able to open up new treatment scenarios.

Therefore, there is a strong need for predictive markers of neurodegenerative diseases, in particular, AD and FTD. It is important to have markers that can be used on individuals with ongoing neurodegenerative processes, but for whom cognitive disorders are not yet manifest, so that prevention and/or treatment protocols can be initiated in a timely manner.

### Description

The authors of the present invention have surprisingly demonstrated that miR-190b-5p (mature sequence of **hsa-miR-190b-5p** publicly available in the miRBase database (Accession ID: MIMAT0004929 accessible directly at the link: https://www.mirbase.orq/results/?query=hsa-miR-190b-5p) is increased in NDEVs isolated from the plasma of patients with AD and/or FTD, compared with nondemented subjects, patients with other dementias, and patients with non-neurodegenerative CNS diseases.

Advantageously, said marker is present in NDEVs isolated from plasma.

It therefore forms an object of the present invention to provide an in vitro method for early detection of cognitive decline, where said method includes:
- Making a plasma sample available from a subject;
- Extracting NDEVs
- Measure, in said sample, the expression levels of miR-190b-5p

Where increased values of said marker compared to controls is indicative of a neurodegenerative process of AD and/or FTD type.

Plasma samples are from prodromal patients, having mild or no overt symptoms, but having positive biomarkers at cerebrospinal fluid analysis or PET with specific neurodegenerative tracers, demonstrating a pathogenic process occurring in the brain. The subjects develop full-blown neurodegeneration typically within 6 months - 1year.

### Description of Figures

**Figure 1****:** miRNA analysis using microRNA primers. The data shown are normalized to the endogenous control (snU6RNA available at: https://www.ensembl.org/Homo sapiens/Gene/Summary?g=ENSG000 00206625;r=15:67839939-67840045;t=ENST00000383898 for miR-190b-5p) and show the relative levels of miR-190b-5p measured in RNA isolated by NDEV from blood of subjects who, one year after measurement, then showed the onset of symptomatology typical of the indicated diseases.
**Figure 2****:** miRNA analysis using microRNA primers as described in Figure 1. Confirmatory data on a cohort of 30 patients per group and 40 controls.

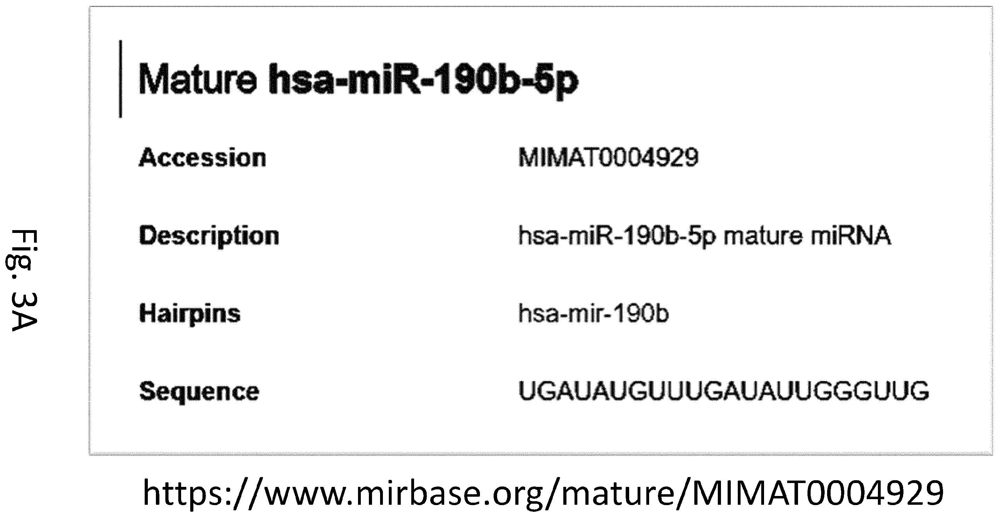

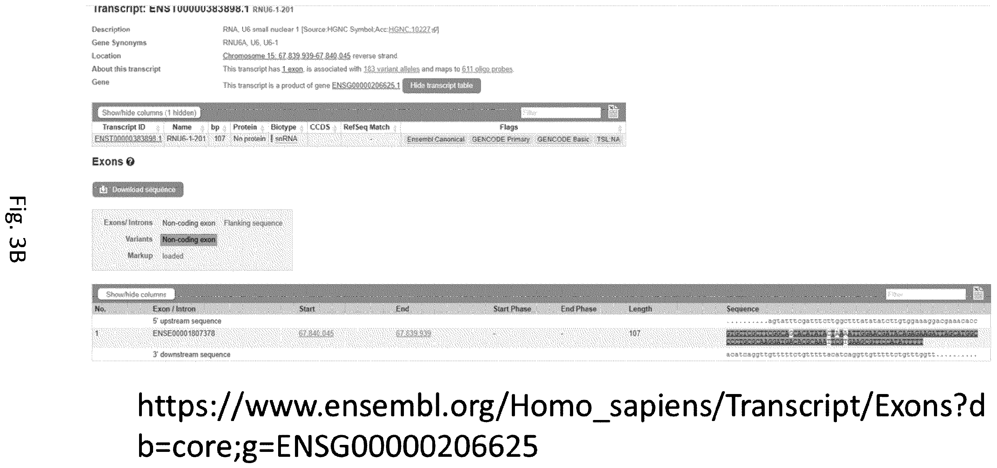

**Figure 3****:** A) miR-190b-5p, B) snU6RNA database outputs.

The following examples are intended to better illustrate the invention. They are not intended to be in any way limiting of the same, the scope of which is defined by the claims

### Examples

### Plasma collection and isolation of NDEVs

The plasma fraction was isolated by centrifugation from whole blood samples.

EV were isolated by methods known in the art (Visconti et al. GeroScience 2023, 45:1557-1571). Briefly, thrombin was added to aliquots of plasma to induce clot formation and subsequent removal of fibrin and protein; after centrifugation, the supernatant was transferred to clean tubes and added Exoquick (System Biosciences Inc CA) for precipitation, by centrifugation, of EVs.

From the total EVs, NDEVs were selected by immunoprecipitation with anti-CD171 neuronal enrichment antibody.

NDEVs were obtained from the following cohorts of subjects. Note that by control subjects, or subjects with one of the listed conditions, we mean here samples of subjects who at day 0, the day of sample collection, had no symptoms associated with any type of dementia. One year later, the same subjects were monitored and classified as listed below, according to the symptomatology that had occurred in the meantime.
- Control (n=11)
- AD (n=11)
- FTD (n=11)
- Lewy Body Dementia (LBD, n=11).

Subjects with dementia of non-neurodegenerative origin were also considered, divided into the following cohorts:
- Vascular dementia (VaD, n=11)

Demyelinating diseases:
- Multiple sclerosis (MS, n=11)

### Isolation and characterization of miRNAs from NDEVs

Total RNA was isolated from NDEV as known in the art (Serpente M. et al. Cell 2020, 9, 1443) and then analyzed with Agilent 2100 Bioanalyzer and RNA 6000 pico (Agilent Technologies, CA, USA) to determine its concentration, purity, and integrity.

Total RNA has also been isolated from serum as known in the art (Serpente M. et al. Cell 2020, 9, 1443)

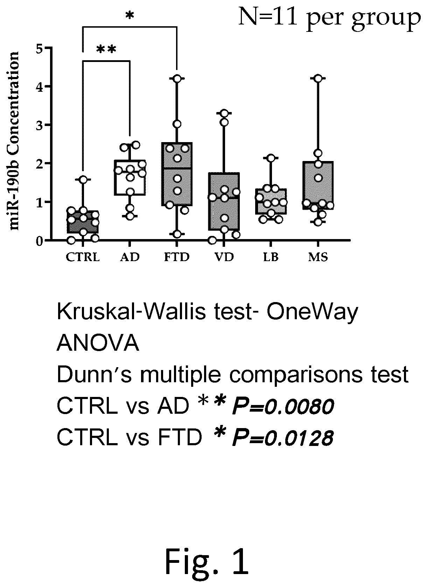

Total RNA, isolated from NDEV or serum, was then back-transcribed and the resulting cDNA preamplified before exposing it to the *real-time* PCR step. *Real-time* PCR was conducted with Droplet Digital PCR QX200, BioRad using the specific primer EVA green YP00206031 - hsa-miR-190b-5p miRCURY LNA miRNA PCR Assay (Qiagen). The results obtained are shown in Figure 1.

The observed increase in miR-190b-5p levels in subjects who were later shown to undergo onset of AD and FTD compared with control subjects is evident. Although to a lesser extent, an increase is also observed in samples from subjects who later went on to experience LBD onset. No increase over control was observed in isolated samples from subjects who later went on to experience dementia not associated with neurodegeneration, (VaD) or non-neurodegenerative CNS diseases, such as Multiple Sclerosis.

Confirming that the observation of the levels of miR-190b-5p contained in NDEVs reflects a neuron-derived event, we report that the measured values of circulating miR-190b-5 then went on to neurodegeneration and control subjects, while the levels of miR-190b-5p contained in NDEVs of the same in serum showed no difference between subjects who are subjects showed that significant increase compared with control values, again measured in NDEVs from healthy subjects, who did not show onset of neurodegenerative pathology.

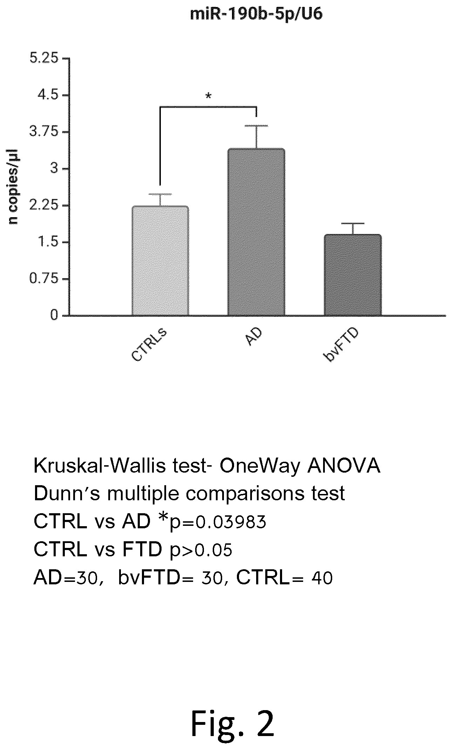

This conclusion was confirmed in a larger cohort of patients as shown in Figure 2.

## Claims

1. Method for predictive diagnosis of dementia associated with neurodegenerative pathology, where said method comprises:
- Isolate neuronal extracellular vesicles (NDEVs) from a plasma sample of a test subject;
- Isolate miRNAs contained in said NDEVs;
- Proceed with a quantitative amplification of miR-190b-5p contained in said miRNAs isolated from said NDEVs and miR-190b-5p contained in a population of miRNAs made available as a control, where levels of miR-190b-5p measured in said miRNAs from said subject in analysis higher than the levels of miR-190b-5p measured in the control are indicative of a forthcoming onset of neurodegeneration-associated dementia.

2. The method according to claim 1, where said increased levels of miR-190b-5p are measured in prodromal patients.

3. The method according to claim 1 or 2, where said dementia associated with neurodegeneration is selected in the group that includes Alzheimer's disease (AD), frontotemporal dementia (FTD), Lewy Body dementia (LBD).

4. The method according to one of claims 1 to 3, where said dementia associated with neurodegeneration is selected in the group consisting of AD and FTD.

5. The method according to one of claims 1 to 4, where said NDEVs are isolated from plasma following precipitation, after removal of fibrin and protein, and subsequent selection with anti-CD171 antibody.
